Europäisches Patentamt

European Patent Office

Office européen des brevets

(11) Publication number: **0 150 544**
**B1**

(12) **EUROPEAN PATENT SPECIFICATION**

(45) Date of publication of patent specification: **13.04.88**

(51) Int. Cl.⁴: **C 07 C 11/09, C 07 C 7/13**

(21) Application number: **84300430.0**

(22) Date of filing: **25.01.84**

(54) Process for the separation of isobutylene from a normal C4 hydrocarbon.

(43) Date of publication of application:
**07.08.85 Bulletin 85/32**

(45) Publication of the grant of the patent:
**13.04.88 Bulletin 88/15**

(84) Designated Contracting States:
**BE DE FR GB IT NL**

(56) References cited:
**EP-A-0 071 477**

**CHEMICAL ABSTRACTS, vol. 99, no. 2, 11th July 1983, page 380, no. 11851y, Columbus, Ohio, US; H. THAMM et al.: "Calorimetric study of the adsorption of n-butane and but-1-ene on a highly dealuminated Y-type zeolite and on silicalite"**

**CHEMICAL ABSTRACTS, vol. 96, no. 8, 22nd February 1982, page 121, no. 54274t, Columbus, Ohio, US**

(73) Proprietor: **UOP Inc.**
**10 UOP Plaza Algonquin & Mt. Prospect Roads**
**Des Plaines Illinois 60016 (US)**

(72) Inventor: **Neuzil, Richard William**
**527 Eldon Place**
**Downers Grove, IL (US)**
Inventor: **Kulprathipanja, Santi**
**3920 Winston Drive**
**Hoffman Estates, IL (US)**

(74) Representative: **Hale, Stephen Geoffrey et al**
**J.Y. & G.W. Johnson Furnival House 14/18 High Holborn**
**London WC1V 6DE (GB)**

Courier Press, Leamington Spa, England.

# 0 150 544

**Description**

It is highly desirable to obtain an isobutylene-rich product, because that butene is useful in gasoline blending and subsequent reactions to produce, for example, butyl rubber and lubricating oil additives. Crystalline aluminosilicates are known in the art to the useful for separating hydrocarbons. Especially, some Type X or Type Y zeolites have been disclosed which have utility in the separation of olefins from paraffinic hydrocarbons and the separation of butene-1 from isobutylene (see US—A—3,723,561 (Priegnitz) and 4,119,678 (Neuzil et al)). Zeolites, however, have the propensity to cause dimerization or even polymerization of olefins and are thus not well suited for use as an adsorbent for the separation of isobutylene from normal $C_4$ hydrocarbons.

Silicalite has been proposed for use as a selective adsorbent for the separation of hydrocarbon mixtures. Thus, EP—A—71477 proposes the use of silicalite to separate normal paraffins of 2—30 carbon atoms per molecule, typically 10—15 or 10—20 carbon atoms per molecule, from feed mixtures also containing cyclic hydrocarbons of more than 6 carbon atoms per molecule and/or branched chain hydrocarbons by selective adsorption of the normal paraffins followed by their recovery using a displacement fluid, preferably a normal paraffin having a boiling point at least 5°C different from the normal paraffin(s) being separated. It is stated there that normally the feed streams used will contain little of no olefins. A calorimetric study of the adsorption of n-butane and butene-1 on silicalite and a highly dealuminated Y-type zeolite is reported in Zeolites, *1983*, 3(2), 95—97 in an article by Thamm et al (abstracted in Chemical Abstracts, 99:11851y (1983), the heat of adsorption for butene-1 on silicalite being less than that for n-butene and butene-1 being reported as being physically adsorbed on silicalite but isomerised on the dealuminated Y-zeolite. Neither reference mentions isobutylene.

We have found that silicalite is able to effect the separation of normal $C_4$ hydrocarbons from isobutylene with substantially complete elimination of the aforementioned undesired side effects of dimerization and polymerization, particularly when pentene-1 is used to displace the normal $C_4$ hydrocarbons from the silicalite.

According to the present invention a process for separating isobutylene from a feed containing isobutylene and at least one normal $C_4$ hydrocarbon comprises contacting the feed at retention conditions with a zeolite-free molecular sieve comprising silicalite to effect selective retention of the normal $C_4$ hydrocarbon, removing the isobutylene from contact with the molecular sieve and recovering the normal $C_4$ hydrocarbon by displacement from the molecular sieve with a displacement fluid comprising pentene-1 at displacement conditions.

In a preferred embodiment, the present invention provides a process for separating isobutylene from a feed containing isobutylene and at least one normal $C_4$ hydrocarbon using a zeolite-free molecular sieve comprising silicalite, which process comprises the steps of: (a) maintaining net fluid flow through a column of the molecular sieve in a single direction, which column contains at least three separate and serially interconnected zones; (b) maintaining a retention zone defined by the feed inlet and raffinate outlet as a downstream boundary; (c) maintaining a purification zone defined by an extract outlet and the feed inlet as a downstream boundary; (d) maintaining a displacement zone defined by a displacement fluid inlet and said extract outlet as a downstream boundary; (e) passing the feed into the retention zone at retention conditions and retaining the normal $C_4$ hydrocarbon and withdrawing a raffinate stream containing isobutylene; (f) passing a displacement material comprising pentene-1 into the displacement zone at displacement conditions and displacing the normal $C_4$ hydrocarbons from the molecular sieve; (g) withdrawing an extract stream comprising the normal $C_4$ hydrocarbon and displacement fluid from the displacement zone; and, (h) periodically advancing through the column of molecular sieve in a downstream direction with respect to fluid flow the feed inlet, raffinate outlet, displacement fluid inlet, and extract outlet to shift zones through the molecular sieve.

In the context of the present invention a "feed mixture" is a mixture containing one or more extract components and one or more raffinate components to be separated by the process. The term "feed stream" indicates a stream of a feed mixture which passes to the molecular sieve used in the process. An "extract component" is a compound or type of compound that is more selectively retained by the molecular sieve while a "raffinate component" is a compound or type of compound that is less selectively retained. In this invention, normal $C_4$ hydrocarbons are extract components and isobutylene is the raffinate component. The term "displacement material or fluid" means a material capable of displacing an extract component. The term "displacing stream" or "displacement fluid input stream" indicates a stream by which displacement material passes to the molecular sieve. The term "raffinate stream" or "raffinate output stream" means a stream through which a raffinate component is removed from the molecular sieve. The composition of the raffinate stream can vary from essentially 100% displacement material to essentially 100% raffinate components. The term "extract stream" or "extract output stream" means a stream through which an extract material which has been displaced by a displacement material is removed from the moelcular sieve. The composition of the extract stream, likewise, can vary from essentially 100% displacement material to essentially 100% extract components.

In a preferred method of practising the process of the invention at least a portion of the extract stream and preferably at least a portion of the raffinate stream from the separation process are passed to separation means, typically fractionators, where at least a portion of displacement material is separated to

2

produce an extract product and a raffinate product. The terms "extract product" and "raffinate product" mean products produced by the process containing, respectively, an extract component and a raffinate component in higher concentrations than those found in the extract stream and the raffinate stream. Although it is possible by the process of this invention to produce a high purity isobutylene product at high recoveries, it will be appreciated that an extract component is never completely adsorbed by the adsorbent, nor is a raffinate component completely nonadsorbed by the adsorbent. Therefore, varying amounts of a raffinate component can appear in the extract stream and, likewise, varying amounts of an extract component can appear in the raffinate stream. The extract and raffinate streams then are further distinguished from each other and from the feed mixture by the ratio of the concentrations of an extract component and a raffinate component appearing in the particular stream. More specifically, the ratio of the concentration of a normal $C_4$ hydrocarbon to that of less selectively retained isobutylene will be lowest in the raffinate stream, next highest in the feed mixture, and the highest in the extract stream. Likewise, the ratio of the concentration of less selectively retained isobutylene to that of a more selectively retained normal $C_4$ hydrocarbon will be highest in the raffinate stream, next highest in the feed mixture, and the lowest in the extract stream.

The term "selective pore volume" of the molecular sieve is defined as the volume of the molecular seive which selectively retains an extract component from the feed mixture. The term "non-selective void volume" of the molecular sieve is the volume of the molecular sieve which does not selectively retain an extract component from the feed mixture. This volume includes the cavities of the molecular sieve which are capable of retaining raffinate components and the interstitial void spaces between molecular sieve particles. The selective pore volume and the non-selective void volume are generally expressed in volumetric quantities and are of importance in determining the proper flow rates of fluid required to be passed into an operational zone for efficient operations to take place for a given quantity of molecular sieve. When molecular sieve "passes" into an operational zone (hereinafter defined and described) employed in one embodiment of this process its non-selective void volume together with its selective pore volume carries fluid into that zone. The non-selective void volume is utilized in determining the amount of fluid which should pass into the same zone in a countercurrent direction to the molecular sieve to displace the fluid present in the non-selective void volume. If the fluid flow rate passing into a zone is smaller than the non-selective void volume rate of molecular sieve material passing into that zone, there is a net entrainment of liquid into the zone by the molecular sieve. Since this net entrainment is a fluid present in the non-selective void volume of the molecular sieve, it in most instances comprises less selectively retained feed components.

Feed stocks which can be utilized in the process of this invention can be derived from any of the refinary processes known to the art. Specifically, the feed stocks include $C_4$ mono-olefinic hydrocarbons such as butene-1, isobutylene, trans-butene-2 and cis-butene-2. The term "butene-2" inculdes both the cis- and trans-isomer configuration of that hydrocarbon. Other materials can be present in the feed stock such as large quantities of paraffinic or napthene substances and in some instances low concentrations of aromatic hydrocarbons other than benzene and other contaminant substances such as the combined sulfur nitrogen compounds. It is preferred, however, to substantially reduce the quantity of components which would contribute to the deactivation of the molecular sieves by blocking off the selective pore passageways to feed stock components.

Specific feed stocks which can be utilized in the process of this invention include a feed stock containing about 35 vol.% butene-1, 32.5 vol.% isobutylene and 32.5 vol.% isobutane. Other feed stock compositions include feed stocks containing approximately 21 vol.% butene-1, 21 vol.% isobutylene, 16 vol.% trans-butene-2, 16 vol.% cis-butene-2 with remaining feed stock components comprising a butane component such as isobutane or normal butane. The feed stock can contain other paraffinic substances having higher molecular weight such as heptane or hexanes or octanes or nonanes or higher molecular weight paraffins. It is preferred to utilize feedstocks having more than 15 vol.% total olefins.

Nonnormal hydrocarbons other than isobutylene, such as isobutane, will be rejected by the silicalite adsorbent and comprise part of the raffinate stream along with the isobutylene. This presents no problem if pure isobutylene is desired, since such hydrocarbons are easily separated from isobutylene by conventional means of distillation. The difficult separation is that of iso-butylene from the normal hydrocarbons, particularly normal olefins, and that separation is accomplished by the present invention.

Desorbent materials or displacement fluids used in various prior art separation processes vary depending upon such factors as the type of operation employed. In connection with the nature and function of desorbents attention is directed to the passage in GB—A—2049667 from page 4 line 45 to page 5 line 7, which is incorporated herein by reference.

We have found that a displacement material comprising pentene-1 is particularly effective in the process of the present invention, especially where operated as an isothermal, isobaric, liquid-phase process. It is usually advantageous to mix pentene-1 with a diluent not selectively retained by the molecular sieve such as isooctane or cyclohexane. Preferably the displacement fluid contains from 10 to 90 LV (liquid volume) % pentene-1.

The molecular sieve to be used in the process of this invention comprises silicalite. As previously mentioned, silicalite is a hydrophobic crystalline silica molecular sieve. Due to its aluminum-free structure, silicalite does not show ion-exchange behaviour, and is hydrophobic and organophilic. Silicalite thus comprises a molecular sieve, but not the hydrated aluminum or calcium silicate comprising a zeolite. Silicalite is uniquely suitable for the separation process of this invention for the presumed reason that its pores are of a size and shape that enable the silicalite to function as a molecular sieve, i.e. accept the molecules of normal $C_4$ hydrocarbons into its channels or internal structure, while rejecting the molecules of isobutylene. A detailed discussion of silicalite may be found in the article "Silicalite, A New Hydrophobic Crystalline Silica Molecular Sieve"; *Nature*, Vol. 271, 9 February 1978, incorporated herein by reference.

The molecular sieve may be employed in the form of a dense compact fixed bed which is alternately contacted with the feed mixture and with displacement material. In the simplest embodiment of the invention, the molecular sieve is employed in the form of a single static bed in which case the process is only semi-continuous. In another embodiment, a set of two or more static beds may be employed in fixed bed contacting with appropriate valving so that the feed mixture is passed through one or more molecular sieve beds, while the displacement materials can be passed through one or more of the other beds in the set. The flow of feed mixture and displacement materials may be either up or down through the displacement fluid. Any of the conventional apparatus employed in static bed fluid-solid contacting may be used. The particles of silicalite molecular sieve preferably have a particle size range of 16—60 mesh (Standard U.S. Mesh).

Silicalite itself is a fine powder and therefore must be bound to obtain the above particle size. An inorganic oxide such as alumina is an acceptable binder, although a fluid-permeable organic polymer is at least as effective, particularly polystyrenedivinylbenzene. The organic polymer offers the further advantage of greater resistance to dissolution of the molecular sieve.

Countercurrent moving bed or simulated moving bed countercurrent flow systems have a much greater separation efficiency than fixed molecular sieve bed systems and are therefore preferred. In the moving bed or simulated moving bed processes, the retention and displacement operations are continuously taking place which allows both continuous production of an extract and a raffinate stream and the continual use of feed and displacement fluid streams. One preferred embodiment of this process utilizes what is known in the art as the simulated moving bed countercurrent flow system. The operating principles and sequence of such a flow system, are described in the passage at page 8 line 53 to page 10 line 10 of GB—A—2049667, which passage is incorporated herein by reference.

Reference can be made to US—A—2,985,589 and to a paper entitled "Continuous Adsorptive Processing—A New Separation Technique" by D. B. Broughton presented at the 34th Annual Meeting of the Society of Chemical Engineers at Tokyo, Japan on April 2, 1969, both incorporated herein by reference, for further explanation of the simulated moving bed countercurrent process flow scheme.

Although both liquid and vapour phase operations can be used in many adsorptive or molecular sieve separation processes, liquid-phase operation is preferred for this process because of the lower temperature requirements and because of the higher yields of extract product that can be obtained with liquid-phase operation over those obtained with vapour-phase operation. Retention conditions suitably include a temperature in the range of from 20°C to 200°C, with 20°C to 100°C being more preferred, and a pressure sufficient to maintain liquid-phase. Displacement conditions suitably include the same range of temepratures and pressures as used for retention conditions.

The size of the units which can utilize the process of this invention can vary anywhere from those of pilot-plant scale (see for example US—A—3,706,812) to those of commercial scale and can range in flow rates from as little as a few cc an hour up to many thousands of litres per hour.

The following example is presented to illustrate the process of this invention.

Example

In this Example the pulse test apparatus described in GB—A—2049667 in the passage at page 6 line 13—46, which passage is incorporated herein by reference, was used to test the ability of inorganic oxide bound silicalite to separate isobutylene from a mixture of isobutylene, n-butane, cis- and trans-butene-2 and butene-1. Two tests were run. In the first test the molecular sieve used was alumina bound silicalite, the displacement fluid was isooctane followed by a hexene-1 and isooctane mixture in the ratio of 60:40, respectively, the column temperature was 50°C and the feed stream pulse was 10 ml of a mixture of the $C_4$ isomers in isooctane. In the second test the molecular sieve used was clay bound silicalite, the displacement fluid was a 50:50 mixture of pentene-1 and cyclohexane (in accordance with the present invention), the column temperature was 60°C and the feed stream pulse was 10 ml of a mixture of the $C_4$ isomers in displacement fluid.

The reason why displacement in the first test was initiated with isooctane was to demonstrate at the onset that the separation was a molecular sieve action, i.e. all $C_4$ components of the feed other than the isobutylene were retained in the silicalite pores while the isobutylene remained in the void spaces from which it was easily flushed. The aspect of the tests of immediate interest was the relative ability of the hexene-1 or pentene-1 in Tests 1 and 2, respectively, to displace the retained $C_4$ components from the silicalite.

The column temperature difference in the two tests is not considered to have a significant effect on the results.

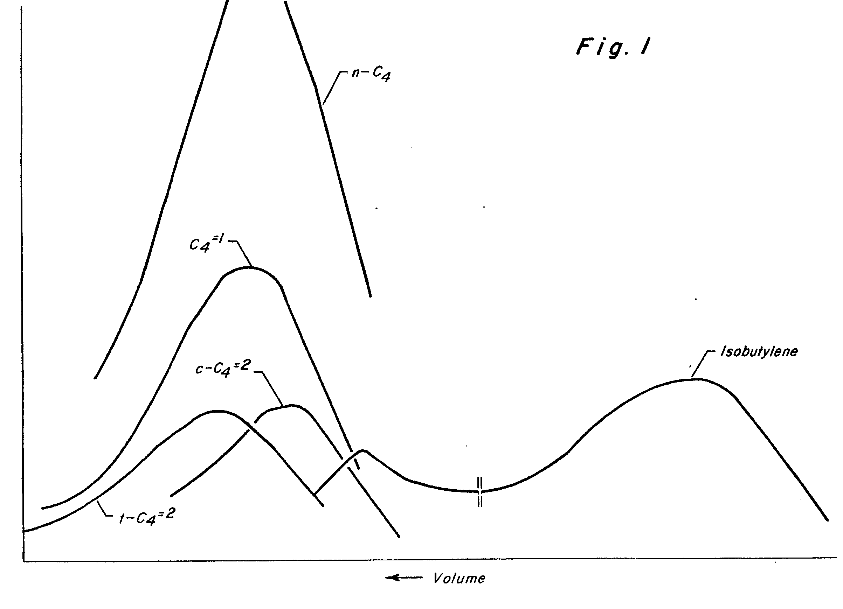

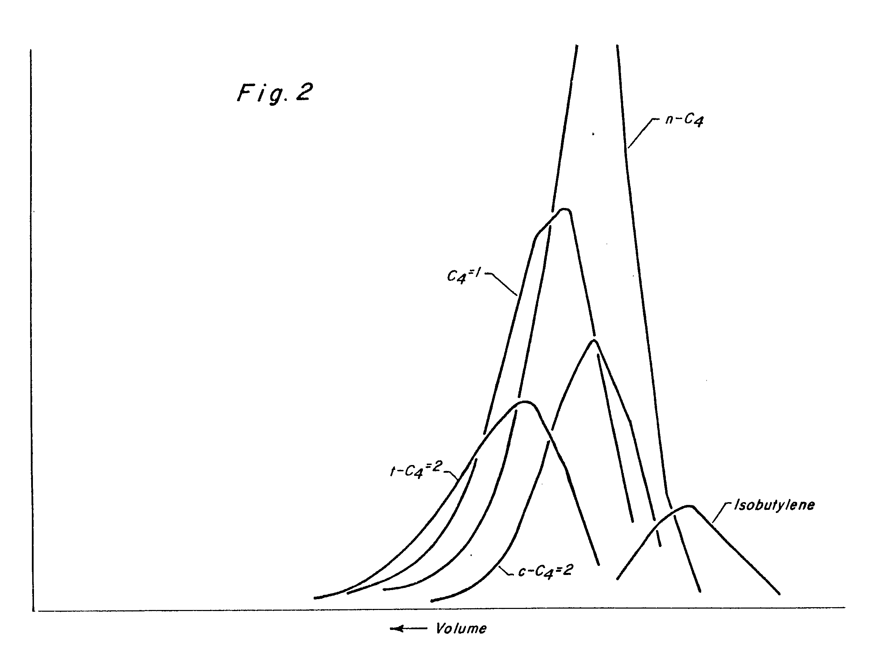

The results obtained from Tests 1 and 2 are presented in the following Table and Figures 1 and 2 of the accompanying drawings which comprise the elution curves generated by pulse Tests 1 and 2, respectively.

4

TABLE

| Test | Retention volume | | | | Half width | | | |
|---|---|---|---|---|---|---|---|---|
| | $n\text{-}C_4$ | $C_4^{=1}$ | $T\text{-}C_4^{=2}$ | $C\text{-}C_4^{=2}$ | $n\text{-}C_4$ | $C_4^{=1}$ | $T\text{-}C_4^{=2}$ | $C\text{-}C_4^{=2}$ |
| 1 | 35.0 | 35.8 | 37.8 | 33.1 | 15.5 | 13.5 | 13.5 | 12.4 |
| 2 | 8.6 | 11.4 | 7.6 | 14.3 | 8.9 | 9.6 | 8.4 | 11.3 |

The isobutylene peak in Figure 1 is shown separate and discontinuous from the other peaks because the isobutylene is eluted with the isooctane pre-flush and prior to the use of displacement fluid.

The data in the Table and the curves in the Figures show the superior rate at which the pentene-1 effects displacement of the retained components (evident by lower retention volumes and half widths) as well as the fact of complete and "clean" displacement of such components achieved by pentene-1 as compared to the severe tailings experienced through use of the hexene-1. The effectiveness of the present invention is thus clearly illustrated.

**Claims**

1. A process for separating isobutylene from a feed containing isobutylene and at least one normal $C_4$ hydrocarbon comprising contacting the feed at retention conditions with a selective adsorbent to effect selective retention of the normal $C_4$ hydrocarbon(s), removing said isobutylene from contact with said selective adsorbent, and recovering the normal $C_4$ hydrocarbon(s) by displacement from said selective adsorbent with a displacement fluid at displacement conditions, characterised in that the selective adsorbent is a zeolite-free molecular sieve comprising silicalite and the displacement fluid comprises pentene-1.

2. A process as claimed in claim 1, characterised in that it comprises the steps of:

(a) maintaining net fluid flow through a column of the selective adsorbent in a single direction, which column contains at least three separate and serially interconnected zones;

(b) maintaining in the column a retention zone extending between a feed inlet and a raffinate outlet at its upstream and downstream boundaries respectively;

(c) maintaining in the column a purification zone extending between an extreact outlet and the feed intlet at its upstream and downstream boundaries respectively;

(d) maintaining in the column a displacement zone extending between a displacement fluid inlet and the extraact outlet at its upstream and downstream boundaries respectively;

(e) passing the feed into the retention zone via the feed inlet at retention conditions and selectively retaining the normal $C_4$ hydrocarbon(s) and withdrawing via the raffinate outlet a raffinate stream containing isobutylene;

(f) passing a displacement fluid into the displacement zone via the displacement fluid inlet at displacement conditions and displacing said normal $C_4$ hydrocarbon(s) from the selective adsorbent;

(g) withdrawing an extract stream comprising the normal $C_4$ hydrocarbon(s) and displacement fluid from the displacement zone via the extract outlet; and

(h) periodically advancing through the column of selective adsorbent in a downstream direction with respect to fluid flow the feed inlet, raffinate outlet, displacement fluid inlet, and extract outlet to shift the zones through the selective adsorbent.

3. A process as claimed in claim 2, characterised in that the raffinate stream is passed to a separation means wherein the displacement fluid is removed from the stream to produce substantially pure isobutylene product.

4. A process as claimed in claim 2 or 3, characterised in that a buffer zone is maintained in the column immediately upstream from the displacement zone, extending between the raffinate outlet and the displacement fluid inlet at its upstream and downstream boundaries respectively.

5. A process as claimed in any of claims 1 to 4, characterised in that the retention conditions include a temperature within the range of from 20°C to 200°C and a pressure sufficient to maintain liquid phase.

6. A process as claimed in any of claims 1 to 5, characterised in that the displacement conditions include a temperature within the range of from 20°C to 200°C and a pressure sufficient to maintain liquid phase.

7. A process as claimed in any of claims 1 to 6, characterised in that the displacement fluid comprises pentene-1 in solution with a diluent not selectively retained by the molecular sieve.

8. A process as claimed in claim 7, characterised in that the displacement fluid contains from 10 to 90 LV% pentene-1.

9. A process as claimed in claim 7 or 8, characterised in that the diluent comprises isooctane or cyclohexane.

10. A process as claimed in any of claims 1 to 9, characterised in that the normal $C_4$ hydrocarbon comprises an olefin.

11. A process as claimed in any of claims 1 to 7, characterised in that the molecular sieve comprises silicalite bound with an inorganic oxide.

12. A process as claimed in any of claims 1 to 7, characterised in that the molecular sieve comprises silicalite bound with a fluid-permeable organic polymer.

13. A process as claimed in claim 12, characterised in that the fluid-permeable organic polymer comprises polystyrenedivinylbenzene.

**Patentansprüche**

1. Verfahren zur Abtrennung von Isobutylen aus einem Isobutylen und mindestens einen normalen $C_4$-Kohlenwasserstoff enthaltenden Einsatzmaterial, bei dem man dieses zur selektiven Rückhaltung des normalen $C_4$-Kohlenwasserstoffs bzw. der normalen $C_4$-Kohlenwasserstoffs unter Rückhaltebedingungen mit einem selektiven Adsorptionsmittel in Kontakt bringt, dieses Isobutylen vom Kontakt mit diesem selektiven Adsorptionsmittel entfernt und den bzw. die normalen $C_4$-Kohlenwasserstoff(e) durch Verdrängung aus diesem selektiven Adsorptionsmittel mit einer Verdrängungsflüssigkeit unter Verdrängungsbedingungen zurückgewinnt, dadurch gekennzeichnet, dass als selektives Adsorptionsmittel ein zeolithfreies, aus Silicalit bestehendes Molekularsieb vorliegt und die Verdrängungsflüssigkeit aus Penten-1 besteht.

2. Verfahren nach Anspruch 1, dadurch gekennzeichnet, dass man stufenwiese:

(a) eine Nettoflüssigkeitsströmung in einer einzigen Richtung durch eine Säule mit dem selektiven Adsorptionsmittel aufrecht erhält, welche mindestens drei getrennte und in Reihe geschaltete Zonen aufweist,

(b) in der Säule eine Rückhaltezone aufrechterhält, die sich zwischen einem Einsatzeinlass und einem Raffinatauslass an ihrer stromaufwärtigen bzw. stromabwärtigen Begrenzung erstreckt,

(c) in der Säule eine Reinigungszone aufrechterhält, welche sich zwischen einem Extraktauslass und dem Einsatzeinlass an ihrer stromaufwärtigen bzw. stromabwärtigen Begrenzung erstreckt,

(d) in der Säule eine Verdrängungzone aufrechterhält, die sich zwischen einem Verdrängungsflüssigkeitseinlass und dem Extraktauslass an ihrer stromaufwärtigen bzw. stromabwärtigen Begrenzung erstreckt,

(e) das Einsatzmaterial über den Einsatzeinlass in die Rückhaltezone unter Rückhaltebedingungen einleitet, den bzw. die normalen $C_4$-Kohlenwasserstoff(e) selektiv zurückhält und über den Raffinatauslass einen Isobutylen enthaltenden Raffinatstrom abzieht,

(f) eine Verdrängungsflüssigkeit über den Verdrängungsflüssigkeitseinlass in die Verdrängungszone unter Verdrängungsbedingungen einleitet und besagte(n) normale(n) $C_4$-Kohlenwasserstoff(e) aus dem selektiven Adsorptionsmittel verdrängt,

(g) einen aus dem bzw. den normalen $C_4$-Kohlenwasserstoff(en) und der Verdrängungsflüssigkeit bestehenden Extraktstrom aus der Verdrängungszone über den Extraktauslass abzieht und

(h) von Zeit zu Zeit den Einsatzeinlass, den Raffinatauslass, den Verdrängungsflüssigkeitseinlass und den Extraktauslass durch die Säule aus selektivem Adsorptionsmittel stromabwärts bezüglich der Flüssigkeitsströmung vorschiebt, um die Zonen durch das selektive Adsorptionsmittel zu schieben.

3. Verfahren nach Anspruch 2, dadurch gekennzeichnet dass der Raffinatstrom zu einer Trennvorrichtung geleitet wird, worin die Verdrängungsflüssigkeit zur Erzeugung weitgehend reinen Isobutylenprodukts aus dem Strom entfernt wird.

4. Verfahren nach Anspruch 2 oder 3, dadurch gekennzeichnet, dass man in der Säule unmittelbar stromaufwärts der Verdrängungzone eine Pufferzone aufrechterhält, die sich zwischen dem Raffinatauslass und dem Verdrängungsflüssigkeitseinlass an ihrer stromaufwärtigen bzw. stromabwärtigen Begrenzung erstreckt.

5. Verfahren nach einem der Ansprüche 1 bis 4, dadurch gekennzeichnet, dass die Rückhaltebedingungen eine Temperatur im Bereich von 20°C bis 200°C und einen zur Aufrechterhaltung einer flüssigen Phase ausreichenden Druck beinhalten.

6. Verfahren nach einem der Ansprüche 1 bis 5, dadurch gekennzeichnet, dass die Verdrängungsbedingungen eine Temperatur im Bereich von 20°C bis 200°C und einen zur Aufrechterhaltung einer flüssigen Phase ausreichenden Druck beinhalten.

7. Verfahren nach einem der Ansprüche 1 bis 6, dadurch gekennzeichnet, dass die Verdrängungsflüssigkeit aus Penten-1 in Lösung mit einem von dem Molekularsieb nicht selektiv zurückgehaltenen Verdünnungsmittel besteht.

8. Verfahren nach Anspruch 7, dadurch gekennzeichnet, dass die Verdrängungsflüssigkeit 10 bis 90% Penten-1 bezogen auf das Lösungsvolumen enthält.

9. Verfahren nach Anspruch 7 oder 8, dadurch gekennzeichnet, dass das Verdünnungsmittel aus Isooctan oder Cyclohexan besteht.

10. Verfahren nach einem der Ansprüche 1 bis 9, dadurch gekennzeichnet, dass der normale $C_4$-Kohlenwasserstoff aus einem Olefin besteht.

11. Verfahren nach einem der Ansprüche 1 bis 7, dadurch gekennzeichnet, dass das Molekularsieb aus mit einem anorganischen Oxid gebundenen Silicalit besteht.

12. Verfahren nach einem der Ansprüche 1 bis 7, dadurch gekennzeichnet, dass das Molekularsieb aus mit einem für. Flüssigkeiten durchlässigen organischen Polymeren gebundenen Silicalit besteht.

13. Verfahren nach Anspruch 12, dadurch gekennzeichnet, dass das für Flüssigkeiten durchlässige organische Polymer aus Polystyroldivinylbenzol besteht.

## Revendications

1. Un procédé pour la séparation de l'isobutylène d'un courant d'alimentation contenant de l'isobutylène et au moins un hydrocarbure normal en $C_4$, comprenant la mise en contact de la charge d'alimentation dans des conditions de rétention avec un adsorbent sélectif pour effectuer la rétention sélective de l'hydrocarbure normal (des hydrocarbures normaux) en $C_4$, le retrait dudit isobutylène du contact avec ledit adsorbant sélectif, et la récupération de l'hydrocarbure normal (des hydrocarbures normaux) en $C_4$ par déplacement dudit adsorbent sélectif dans un fluide de déplacement dans des conditions de déplacement, caractérisé en ce que l'adsorbent sélectif est un tamis moléculaire, dépourvu de zéolithes, comprenant de la silicalite, et en ce que le fluide de déplacement comprend du pentène-1.

2. Un procédé selon la revendication 1, caractérisé en ce qu'il comprend les étapes suivantes:

(a) maintenir un écoulement net de fluide à travers une colonne de l'adsorbant sélectif dans un sens unique, laquelle colonne contient au moins trois zones séparées et communiquant en série;

(b) maintenir dans la colonne une zone de rétention qui s'étend entre une entrée de charge d'alimentation et une sortie de raffinat à ses limites respectivement amont et aval;

(c) maintenir dans la colonne une zone de purification qui s'étend entre une sortie d'extrait et l'entrée de charge d'alimentation à ses limites respectivement amont et aval;

(d) maintenir dans la colonne une zone de déplacement qui s'étend entre une entrée de fluide de déplacement et la sortie d'extrait à ses limites respectivement amont et aval;

(e) faire passer la charge d'alimentation dans la zone de rétention par l'entrée de charge d'alimentation dans des conditions de rétention, et retenir sélectivement l'hydrocarbure normal (les hydrocarbures normaux) en $C_4$ et soutirer par la sortie de raffinat un courant de raffinat contenant de l'isobutylène;

(f) faire passer un fluide de déplacement dans la zone de déplacement par l'entrée de fluide de déplacement dans des conditions de déplacement et déplacer ledit hydrocarbure normal (lesdits hydrocarbures normaux) en $C_4$ de l'adsorbant sélectif;

(g) retirer un courant d'extrait comprenant l'hydrocarbure normal (les hydrocarbures normaux) en $C_4$ et le fluide de déplacement de la zone de déplacement par la sortie d'extrait; et

(h) faire avancer périodiquement à travers la colonne d'adsorbant sélectif, dans un sens vers l'aval par rapport à l'écoulement de fluide, l'entrée de charge d'alimentation, la sortie de raffinat, l'entrée de fluide de déplacement, et la sortie d'extrait, pour déplacer les zones à travers l'adsorbant sélectif.

3. Un procédé selon la revendication 2, caractérisé en ce que l'on fait passer le courant de raffinat dans un moyen de séparation dans lequel on retire le fluide de déplacement du courant, pour obtenir le produit isobutylène sensiblement pur.

4. Un procédé selon la revendication 2 ou 3, caractérisé en ce que l'on maintient une zone tampon dans la colonne, immédiatement en amont de la zone de déplacement, zone qui s'étend entre l'entrée de fluide de déplacement et la sortie de raffinat à ses limites respectivement amont et aval.

5. Un procédé selon l'une quelconque des revendications 1 à 4, caractérisé en ce que les conditions de rétention comportent une température comprise dans l'intervalle de 20°C à 200°C et une pression suffisante pour maintenir la phase liquide.

6. Un procédé selon l'une quelconque des revendications 1 à 5, caractérisé en ce que les conditions de déplacement comportent une température comprise dans l'intervalle de 20°C à 200°C et une pression suffisante pour maintenir une phase liquide.

7. Un procédé selon l'une quelconque des revendications 1 à 6, caractérisé en ce que le fluide de déplacement comprend du pentène-1 en solution dans un diluant qui n'est pas retenu sélectivement par le tamis moléculaire.

8. Un procédé selon la revendication 7, caractérisé en ce que le fluide de déplacement contient de 10 à 90% en VL de pentène-1.

9. Un procédé selon la revendication 7 ou 8, caractérisé en ce que le diluant comprend de l'isooctane ou du cyclohexane.

10. Un procédé selon l'une quelconque des revendications 1 à 9, caractérisé en ce que l'hydrocarbure normal en $C_4$ comprend une oléfine.

11. Un procédé selon l'une quelconque des revendications 1 à 7, caractérisé en ce que le tamis moléculaire comprend de la silicalite liée à un oxyde minéral.

12. Un procédé selon l'une quelconque des revendications 1 à 7, caractérisé en ce que le tamis moléculaire comprend de la silicalite liée à un polymère organique perméable aux fluides.

13. Un procédé selon la revendication 12, caractérisé en ce que le polymère organique perméable aux fluides comprend du polystyrènedivinylbenzène.

*Fig. 1*

n–C$_4$

C$_4$=1

c–C$_4$=2

t–C$_4$=2

Isobutylene

Volume

0 150 544

Fig. 2